# EUROPÄISCHE PATENTSCHRIFT

(11) **EP 0 295 540 B1**
(45) Veröffentlichungstag und Bekanntmachung des Hinweises auf die Patenterteilung: **11.05.1994**
(21) Anmeldenummer: 88109064.1
(22) Anmeldetag: 07.06.1988
(51) Int. Cl.: C07K 1/04

(54) **Allylische Seitenketten enthaltendes Festphasensystem, Verfahren zu seiner Herstellung und seine Verwendung in Festphasenreaktionen**
Solid-phase system containing allylic chains, process for its preparation and its use in solid-phase reactions
Système en phase solide contenant des chaînes allyliques, son procédé de préparation et son utilisation dans les réactions en phase solide

(30) Priorität: 19.06.1987 DE 3720269; 05.02.1988 DE 3803545
(43) Veröffentlichungstag der Anmeldung: 21.12.1988
(73) Patentinhaber: ORPEGEN Medizinisch-Molekularbiologische Forschungsgesellschaft m.b.H, 69115 Heidelberg (DE)
(72) Erfinder: Kunz, Horst, Dr., D-6500 Mainz-Drais (DE); Dombo, Berthold, D-6200 Wiesbaden-Medenbach (DE)
(74) Vertreter: Huber, Bernhard, Dipl.-Chem.

(56) Entgegenhaltungen:
- CHEMICAL ABSTRACTS, Band 105, Nr. 1, 7. Juli 1986, Seite 638, Zusammenfassung Nr. 6797y, Columbus, Ohio, US; D. BELLOF et al.: "A new phenacyl-type handle for polymer supported peptide synthesis", & CHIMIA 1985, 39(10), 317-20
- CHEMICAL ABSTRACTS, Band 93, Nr. 18, 3. November 1980, Seite 21, Zusammenfassung Nr. 168898t, Columbus, Ohio, US; & JP-A-80 69 603 (FUJISAWA PHARMACEUTICAL CO., LTD) 26-05-1980
- CHEMICAL ABSTRACTS, Band 109, Nr. 7, 15. August 1988, Seite 731, Zusammenfassung Nr. 55223x, Columbus, Ohio, US; H. KUNZ et al.: "Solid phase synthesis of peptide and glycopeptide polymers with allylic anchor groups", & ANGEW. CHEM. 1988, 100(5), 732-4

## Beschreibung

Die Erfindung betrifft Festphasensysteme, die allylische Seitenketten enthalten, Verfahren zu ihrer Herstellung und ihre Verwendung in Festphasenreaktionen, insbesondere zur Festphasensynthese von Peptiden, Glycopeptiden oder Proteinen.

Die gezielte Synthese von Peptiden gewinnt angesichts der zunehmenden Anforderungen an Pharmaka, Lebensmittelzusatzstoffe und andere Wirkstoffe hinsichtlich der Selektivität der Wirkung, der Verträglichkeit und der biologischen Abbaubarkeit wieder stark an Bedeutung. Trotz der nunmehr hoch entwickelten gentechnologischen Techniken gilt dies auch für die chemische Synthese von Peptiden, durch die allein z.B. Peptide mit nicht-natürlichen Bausteinen und Strukturelementen zugänglich sind.

Für den chemischen Aufbau von Peptiden bedeutete die Einführung der Festphasensynthese nach R.B. Merrifield (R.B. Merrifield, J. Am. Chem. Soc. 85, 2149 (1963)) einen großen Fortschritt. Das gilt trotz der inzwischen erkannten Probleme, die bei diesen Festphasen-Peptidsynthesen hinsichtlich der Reinheit der synthetisierten Produkte immer wieder auftreten.

Als C-terminale Schutz- und Ankergruppen dienen in den Festphasensynthesen substituierte Benzylester, die die Abspaltung der aufgebauten Peptide vom polymeren Träger unter mehr oder weniger stark sauren Bedingungen erlauben. Die sauren Abspaltungsbedingungen - in der klassischen Merrifield-Synthese wendet man Bromwasserstoff in verschiedenen Lösungsmitteln oder Fluorwasserstoff an - haben den Nachteil, daß dabei unerwünschte Nebenreaktionen, wie Transpeptidierungen oder -alkylierungen, auftreten können. Die Synthese von Glycopeptiden ist in dieser Weise kaum durchführbar, weil die empfindlichen glycosidischen Bindungen dieser Moleküle unter sauren Bedingungen gespalten oder anomerisiert werden (zum Stand der Glycopeptidsynthese vgl. z.B. H. Kunz (Angewandte Chemie, 99 (1987); Angew. Chemie, Int. Ed. Engl. 26 (1987) 294).

Aufgabe der vorliegenden Erfindung ist die Bereitstellung von Festphasensystemen, insbesondere zur Festphasensynthese von Peptiden und Glycopeptiden, bei denen die Peptide und Glycopeptide vom polymeren Träger selektiv und unter solchen milden Bedingungen abgespalten werden können, bei denen keine Spaltung der glycosidischen Bindungen oder Anomerisierungen auftreten.

Gegenstand der Erfindung ist ein allylische Seitenketten enthaltendes Festphasensystem gemäß Anspruch 1, in dem die allylischen Seitenketten an ein festes Trägermaterial gebunden sind, mit der allgemeinen Formel 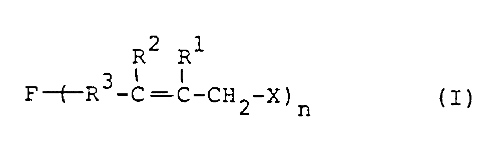
worin F ein festes Trägermaterial bedeutet, R¹ und R² gleich oder verschieden sind und Wasserstoff eine Alkylgruppe mit 1 bis 7 Kohlenstoffatomen, eine substituierte oder unsubstituierte ein- oder zweikernige Arylgruppe oder Halogen bedeuten und zwei Alkylsubstituenten zusammen mit einer Arylgruppe in R¹ oder R² auch ein System aus 2 oder mehreren Ringen bilden können, R³ eine Verknüpfungsgruppierung (Spacer) bedeutet, X Hydroxyl, Halogen, eine Sulfonatgruppe, eine Phosphonatgruppe oder eine Acyloxygruppe bedeutet, wobei Acyl in der Acyloxygruppe den Rest einer aliphatischen Carbonsäure mit 1 bis 7 Kohlenstoffatomen darstellt oder den Rest RCO-darstellt, der den geschützten oder ungeschützten Rest einer Aminosäure, eines Peptids, Glycopeptids, Nucleotids, einer Hydroxycarbonsäure, einer Dicarbonsäure oder einer Tricarbonsäure bedeutet, der über die Carbonylgruppe gebunden ist, und n die Zahl der auf dem Trägermaterial gebundenen Seitenketten ist. Zweckmäßige Ausgestaltungen davon sind Gegenstand der Ansprüche 2 bis 6.

Die Alkylgruppe R¹ oder R² kann geradkettig oder verzweigt sein; vorzugsweise ist die Alkylgruppe eine Niederalkylgruppe mit 1 bis 3 Kohlenstoffatomen.

Eine Arylgruppe in R¹ oder R² ist eine substituierte oder unsubstituierte ein- oder zweikernige Arylgruppe, wie z.B. Naphthyl-(1)- oder (2)-, und insbesondere Phenyl. Die Arylgruppe kann durch einen oder mehrere Substituenten, wie Niederalkyl und/oder Halogen substituiert sein, ist aber vorzugsweise unsubstituiert. Zwei Alkylsubstituenten können zusammen mit der Arylgruppe auch ein System aus zwei oder mehreren Ringen bilden, wie z.B. Tetrahydronaphthalin.

Halogen kann Fluor, Chlor, Brom oder Jod sein, und ist vorzugsweise, insbesondere in der Bedeutung von X, Chlor oder Brom.

Der Rest R³ stellt eine Verknüpfungsgruppe (Spacer, Links) dar und ist z.B. eine der bisher in der Festphasen-Technik (Merrifield-Technik) verwendete Spacer-Gruppierung (z.B. CASET, CAMET; vgl. z.B. Römpps Chemie-Lexikon, 8. Auflage, Seite 2543). Die Art der Gruppe R³ richtet sich dabei insbesondere auch nach dem weiter unten beschriebenen Verfahren zur Herstellung der Festphasensysteme der Formel (I), insbesondere nach der Art des Trägermaterials, und der auf dem Trägermaterial und der die Allylgruppe enthaltenden Verbindung vorhandenen funktionellen Gruppen A und B. Die Gruppe R³ kann z.B. eine Alkylen-, Aralkylen- oder Arylen-Gruppierung sein, die an einem oder beiden Enden polare Gruppen enthalten kann. Für Polystyrol als Trägermaterial ist R³ z.B. die Gruppierung -CH₂-NHCO-.

In einer Acyloxygruppe X, in der Acyl den Rest einer aliphatischen Carbonsäure darstellt, ist Acyl vorzugsweise der Säurerest einer aliphatischen Carbonsäure mit 1 bis 7, vorzugsweise 1 bis 4 Kohlenstoffatomen, z.B. Formyl, Acetyl und Propionyl.

Für das feste Trägermaterial geht man vorzugsweise von einem solchen Trägermaterial aus, das funktionelle Gruppen aufweist, die sich gut für die Umsetzung mit der die Allylgruppierung tragenden Verbindung eignen, wobei sich die Spacergruppierung R³ während dieser Umsetzung ausbilden oder auch bereits teilweise oder ganz Bestandteil (Substituent) des festen Trägermaterials sein kann.

Das Trägermaterial ist vorzugsweise ein organisches oder anorganisches Polymeres, wie z.B. ein synthetisches, halbsynthetisches oder natürliches Polymeres; solche Polymere sind z.B. vernetzte Polyacrylamide, Methacrylate, Dextrane (z.B. Sephadex®), Cellulose, und insbesondere Polystyrol. Das Trägermaterial kann aber auch aus einem festen Basismaterial bestehen, das mit einem für die Verknüpfung mit den Allylseitenketten geeigneten Material überzogen ist, wie z.B. mit einem geeigneten Polymeren oder einem vernetzten Protein. Das Basismaterial kann z.B. Glas, Kieselgel oder auch ein Kunstharz sein. Für das Trägermaterial und als Überzug geeignete organische Polymere sind vorzugsweise Polyacrylamide, Polyethylenglycol, und insbesondere Polystyrol.

Zur Ausbildung einer geeigneten Spacergruppierung R³ kann es zweckmäßig sein, z.B. ein mit Aminomethylgruppen substituiertes Polystyrol einzusetzen; im erfindungsgemäßen Festphasensystem sind die Aminomethylgruppierungen dann vorzugsweise durch die Gruppierung -CO-(CH₂)ₓ-C(R²)=C(R¹)-CH₂-X substituiert, worin x 0 oder eine ganze Zahl, vorzugsweise von 1 - 10 und insbesondere von 1 - 7, bedeutet.

Gegenstand der Erfindung ist auch ein Verfahren gemäß Anspruch 8 zur Herstellung von erfindungsgemäßen Verbindungen der allgemeinen Formel (I), worin X Hydroxyl, Halogen, eine Sulfonatgruppe, eine Phosphonatgruppe oder eine Acyloxygruppe, worin Acyl den Rest einer aliphatischen Carbonsäure darstellt, bedeutet, das dadurch gekennzeichnet ist, daß man ein festes Trägermaterial, das zur Verknüpfung mit der Allylgruppierung C(R²)=C(R¹)-CH₂-X geeignete funktionelle Gruppen A enthält, mit einer Verbindung B-C(R²)=C(R¹)-CH₂-X umsetzt, worin A und B Atomgruppierungen bedeuten, die unter Kondensation und/oder Addition und Ausbildung einer Verknüpfung zwischen festem Trägermaterial und Allylgruppierung miteinander reagieren.

Zweckmäßige Ausgestaltungen dieses Verfahrens sind Gegenstand der Ansprüche 9 und 10.

Bei den Resten A und B handelt es sich um solche Gruppierungen, die unter Kondensation und/oder Addition und Ausbildung einer Verknüpfung zwischen dem Trägermaterial und der Allylgruppierung reagieren. Solche Gruppen sind vorzugsweise die in Kondensations- und Additionsreaktionen üblicherweise verwendeten Gruppen, z.B. Aminogruppen, z.B. in Form einer Aminomethylgruppe, Halogen, Estergruppierungen, Nitrilgruppen usw. Die Kondensations- und/oder Additionsreaktionen können in der Regel auf an sich bekannte Weise, z.B. unter Abspaltung von Wasser, Halogenwasserstoff usw. durchgeführt werden.

In einer bevorzugten Ausführungsform wird z.B. ein Aminomethylgruppen (Gruppe A) enthaltendes Polystyrol mit einer terminal allylisch substituierten ungesättigten Carbonsäure oder einem Carbonsäurederivat, oder mit den 1,3-Allylisomeren davon, unter Bildung einer Amidgruppierung umgesetzt. Eine allylisch ungesättigte Carbonsäure ist z.B. 4-Brom-Crotonsäure (B=Br), in der nach der Umsetzung unter Amidgruppenbildung die COOH-Gruppe z.B. in eine -CH₂Br-Gruppe überführt wird (X=Br).

Weitere allylisch ungesättigte Carbonsäuren sind z.B. 4-Halogen-, 4-Hydroxy-, 4-Acyloxy- oder 4-Sulfonyloxy-Crotonsäure.

Ein weiterer Gegenstand der Erfindung ist auch ein Verfahren gemäß Anspruch 11 zur Herstellung von Verbindungen der allgemeinen Formel (I), worin X eine Acyloxygruppe bedeutet, in der der Acylrest RCO- den geschützten oder ungeschützten Rest einer Aminosäure, eines Peptids, Glycopeptids, Nucleotids, insbesondere Oligonucleotids, einer Hydroxycarbonsäure, Dicarbonsäure oder Tricarbonsäure bedeutet, das dadurch gekennzeichnet ist, daß man ein Festphasensystem der allgemeinen Formel (I), worin X Hydroxy, Halogen, eine Sulfonatgruppe, eine Phosphonatgruppe oder eine Acyloxygruppe, in der Acyl den Rest einer aliphatischen Carbonsäure darstellt, bedeutet, mit N-geschützten Aminosäuren, Peptiden, Glycopeptiden, Nucleotiden, Hydroxycarbonsäuren, Dicarbonsäuren oder Tricarbonsäuren, oder ihren Derivaten oder Salzen umsetzt, und gegebenenfalls die N-terminalen Schutzgruppen abspaltet. Unter einem Peptidrest ist dabei ganz allgemein auch der Rest eines Proteins, z.B. eines Enzyms, zu verstehen.

Die Umsetzung mit den N-geschützten Aminosäuren, Peptiden, Glycopeptiden, Nucleotiden, oder Hydroxycarbonsäuren, Dicarbonsäuren oder Tricarbonsäuren, oder Derivaten oder Salzen davon kann in an sich bekannter Weise erfolgen, z.B. wie in der üblichen Festphasen-Technik (Merrifield-Technik) üblich. Als Schutzgruppen können dabei die üblicherweise verwendeten Schutzgruppen dienen, wie z.B. Z (Cbo, Cbz), Boc, Ddz, Trt, Bpoc (Dpoc), OBu^{t}, OBzl, Nps, Ddz, Fmoc, Msoc, Mch, Dcboc (zu den Abkürzungen vgl. z.B. Eur. J. Biochem. 74 (1977) 1-6).

Die in den erfindungsgemäßen Festphasensystemen und Verfahren verwendeten C-terminalen Schutz(Anker)-Gruppen, über die die Aminosäuren und Peptide, Glycopeptide usw. an den polymeren Träger gebunden sind, gehören zum Allylester-Typ. Allylester können als C-terminale Schutzgruppen in Glycopeptid-, Nucleotid- und Peptidsynthesen selektiv und unter milden, nahezu neutralen Bedingungen von den blockierten Verbindungen abgespalten werden (vgl. H. Kunz, Angew. Chemie 99 (1987) 297). Man erreicht dies durch Edelmetallkatalyse, wie z.B. durch Katalyse mit Verbindungen der Platinmetalle wie Ru, Rh, Pd, Os, Ir, Pt, und insbesondere durch Katalyse mit Rhodium (I)-Verbindungen (vgl. H. Waldmann, H. Kunz, Liebigs Ann. Chem. 1983 1712) oder mit durch Palladium(0)-Verbindungen katalysierte Reaktionen (H. Kunz, H. Waldmann, Angew. Chemie, 96 (1984) 47; Angew. Chemie Int. Ed. engl. 23 (1984) 71; H. Kunz und C. Unverzagt, Angew. Chemie 96 (1984) 426; Angew. Chemie, Int. Ed. Engl. 23 (1984) 436. Durch die Übertragung dieser Deblockierungsmethodik auf die Festphasenpeptid-Synthese wird erfindungsgemäß erreicht, daß die Ablösung der am Träger aufgebauten Peptid-, Glycopeptid- oder Nucleotidkette nun praktisch unter neutralen Bedingungen möglich ist.

Gegenstand der Erfindung ist deshalb auch ein Verfahren gemäß Anspruch 12 zur Synthese von ungeschützten oder partiell geschützten Peptiden, Glycopeptiden, Nucleotiden, Hydroxycarbonsäuren, Dicarbonsäuren oder Tricarbonsäuren, das dadurch gekennzeichnet ist, daß man aus einer Verbindung der allgemeinen Formel (I), worin X einen Acyloxyrest bedeutet, in dem Acyl den geschützten oder ungeschützten Rest eines Peptids, Glycopeptids, Nucleotids, einer Hydroxycarbonsäure, einer Dicarbonsäure oder einer Tricarbonsäure bedeutet, den Rest X in Gegenwart katalytischer Mengen einer Verbindung der Platingruppenmetalle abspaltet.

Die Abspaltung in Gegenwart katalytischer Mengen einer Verbindung der Platingruppenmetalle, vorzugsweise in Gegenwart einer Rhodium(I)-Verbindung und insbesondere in Gegenwart einer Palladium(0)-Verbindung, kann in einem dafür geeigneten Lösungsmittel oder Lösungsmittelsystem durchgeführt werden, z.B. in Tetrahydrofuran, in Gegenwart eines geeigneten Nucleophils, wie z.B. Morpholin, Dimedon, oder einer anderen leicht deprotonierbaren CH-aciden Verbindung. Die Reaktion wird vorzugsweise bei Raumtemperatur durchgeführt. Es wird unter Sauerstoffausschluß gearbeitet.

Aufgrund der außerordentlich milden Abspaltungsbedingungen ist es erfindungsgemäß möglich, die Abspaltung der Peptide, Glycopeptide, Nucleotide und anderen Reste selektiv und ohne Spaltung glycosidischer Bindungen oder von Anomerisierungen oder Isomerisierungen durchzuführen.

Die erfindungsgemäßen Festphasensysteme lassen sich neben ihrer Verwendung in der Festphasen-Technik (Merrifield-Technik) zur Peptidsynthese oder Glycopeptidsynthese auch als Festphasen in anderen Festphasen-Techniken einsetzen, z.B. zur Synthese von Oligonucleotiden, die sich gegebenenfalls enzymatisch weiter verlängern lassen, z.B. zu bestimmten DNA-Sequenzen, zu Sequenzanalysen, aber auch bei Reaktionen, bei denen Enzyme, katalytisch wirkende Lewissäuren, Redoxsysteme und dergleichen durch Immobilisierung infolge Adsorption, Polymerisation, Pfropfung usw. in eine feste Form gebracht werden, was die Dosierung, Wiederaufbereitung und Abtrennung von Reaktionsprodukten erleichtert. Daneben ist auch eine Anwendung in Festbettverfahren möglich (vgl. z.B. Römpps Chemie-Lexikon, 8. Auflage, Seite 1263 und 2543).

Gegenstand der Erfindung ist deshalb auch die Verwendung eines erfindungsgemäßen Festphasensystems der allgemeinen Formel (I), worin X Hydroxyl, Halogen, eine Sulfonatgruppe, eine Phosphonatgruppe oder eine Acyloxygruppe, worin Acyl den Rest einer aliphatischen Carbonsäure darstellt, bedeutet, für Festphasenreaktionen, und insbesondere für eine Festphasensynthese von Peptiden, Glykopeptiden, Nucleotiden oder Proteinen, z.B. von Enzymen.

Nachfolgend werden einige bevorzugte Ausführungsformen von erfindungsgemäßen Festphasensystemen, Verfahren zu ihrer Herstellung und ihrer Verwendung beschrieben.

### Synthese von polymeren Trägern mit allylischen Ankergruppen

Zur Synthese von Polymeren der allgemeinen Formel I mit Seitenketten, die allylische Gruppen tragen, können verschiedene Wege beschritten werden. Das prinzipielle Vorgehen wird an einem kommerziell erhältlichen (Bio-Rad, Bio-Beads S-Xl 1,28 mg Cl₂/g) chlormethylierten Polystyrol 1 gezeigt, das zunächst in bekannter Weise (A.R.Mitchell u.Mitarb., J.Am.Chem.Soc. 98 (1976), 7337) mit Phthalimid-Kalium über das Phthaloyl-amido-Derivat 2 in das Amino-methyl-polystyrol 3 überführt wird. 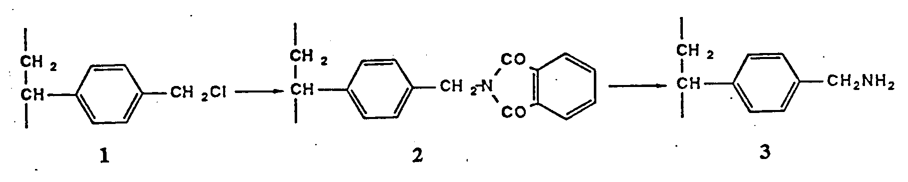
Alternativ kann die Zwischenstufe 2 ebenfalls in bekannter Weise (R.B.Merrifield u.Mitarb. Tetrahedron Lett. 42, 3795 (1976)) aus geeignet vernetztem Polystyrol durch Umsetzung mit N-(Chlormethyl)phthalimid hergestellt werden.
Die Umwandlung von 2 in 3 erreicht man mit Hydrazin in Ethanol (s.A.R.Mitchell et al., o.bez.Literatur).
Das aminomethylierte Polystyrol 3 wird nun, als Beispiel für polymere Träger mit Aminogruppen, in Verbindungen der allgemeinen Formel I umgewandelt, indem es mit Acyl- oder Alkylverbindungen umgesetzt wird, die eine terminale Allyl-halogenid-, -ester- oder -alkohol-Gruppierung tragen. Als Beispiel ist nachfolgend die Umsetzung des Harzes 3 mit 4-Bromcrotonsäure 4 (s.K.Ziegler u. Mitarb., Liebigs Ann.Chem. 551 (1942), 117) beschrieben. Die Verknüpfung erreicht man z.B. mit Hilfe von Dicyclohexylcarbodiimid (DCC) in Gegenwart von 4-Dimethylamino-pyridin (DMAP) (Beispiel 1), oder mit DCC/1-Hydroxy-benzotriazol in CH₂Cl₂ (vgl. W. König und R. Geiger, Chem. Ber. 103 (1970) 788). 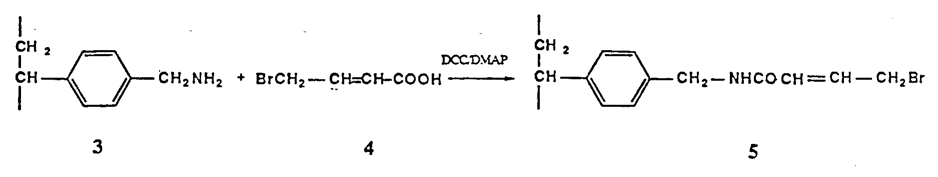
Statt der Brom-crotonsäure 4 können in gleicher Weise längerkettige allylisch substituierte Alken-carbonsäuren eingesetzt werden, z.B. mit der Formel X-CH₂-CH=CH-(CH₂)ₓ-COOH worin x = Br und x z.B. 0 oder 7 ist (im letzten Fall z.B. in Gemisch mit 9-Br-10-undecensäure). Ebenso erhält man in analogen Reaktionen mit hydroxymethylierten Polymeren anstelle von 3 zu 5 analoge Ester.
Die Verbindung 5 ist ein Beispiel für Substanzen der allgemeinen Formel I, worin X = Br. Auf analoge Weise lassen sich ausgehend von einer Verbindung 3 und entsprechende Überführung der Carboxylgruppe oder nachträgliche Überführung von Br in Verbindung 5 Verbindungen der Formel I darstellen, in denen X die Bedeutung Halogen, Hydroxyl, Sulfonat, Phosphonat oder Acyloxy hat, wobei Acyl der Rest einer aliphatischen Carbonsäure ist.

### Ankupplung der C-terminalen Aminosäure an den erfindungsgemäßen Träger

Durch Reaktion der Salze, insbesondere der Cäsiumsalze, von N-geschützten Aminosäuren 6 mit Harzen des Typs 5 werden die C-terminalen Aminosäuren des zu synthetisierenden Peptids oder Glycopeptids an den polymeren Träger angekoppelt. Die dabei gebildeten Kupplungsprodukte, wie 7, entsprechen der allgemeinen Formel I (Beispiel 2 für Alanin). Die Ausbeuten liegen bei 70 - 85 %. Für die folgenden N-geschützten Aminosäuren 6 sind als Ausbeuten z.B. zu erhalten: Boc-Ala: 82 %; Boc-Leu: 73 %; Boc-Ile: 70 %: Z-Ala: 80 %. 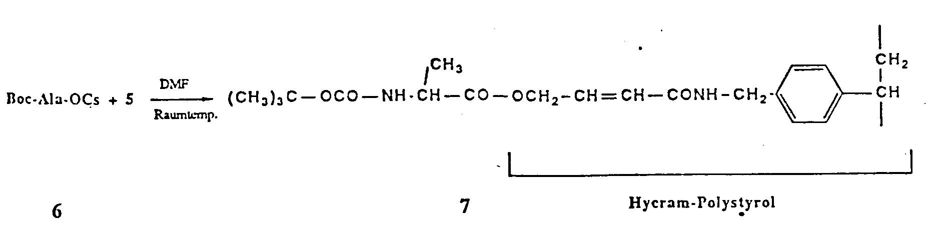
Von der in 7 als (substituierter) Allylester an den Polymeren Träger gebundenen Aminosäure kann die N-terminale Schutzgruppe selektiv abgespalten werden. Im Falle der tert-Butyloxycarbonyl(Boc)-Gruppe (z.B. in 7) gelingt dies durch Behandeln mit Trifluoressigsäure (Beispiel 3). Die Allylester-Bindung an den Träger bleibt völlig stabil. 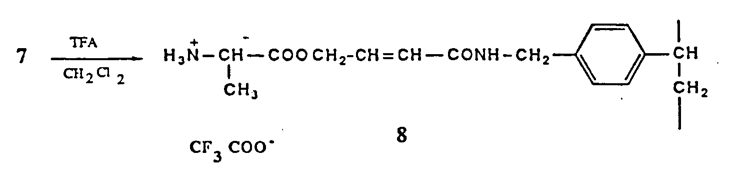
Aus den gebundenen Aminosäure-hydrosalzen, wie 8, wird mit tertiären Basen die freie Aminoverbindung erhalten. Verwendet man statt der Boc-Gruppe unter neutralen oder basischen Bedingungen spaltbare Amino-Schutzgruppen, z.B. die mit Morpholin oder Piperidin/Dichlormethan abspaltbare Fluorenylmethoxycarbonyl (Fmoc)-Gruppe, so werden direkt die freien polymergebundenen Aminoverbindungen gebildet.

### Peptid- und Glycopeptidsynthese am erfindungsgemäßen Träger

An diesen N-deblockierten Substanzen wird nun der Aufbau der Peptidkette nach den in der Festphasen-Peptidsynthese bekannten Methoden (vgl. z.B. R.B.Merrifield, E.Girali et.al., Synthesis 1985, 181; Ch. Birr, "Aspects of the Merrifield Peptide Synthesis" in "Reactivity and Structure Concepts in Organic Chemistry", Bd. 8; Hrsg. K. Hafner et al., Springer-Verlag, Berlin-Heidelberg-New York 1978; J. Lenard, A.B. Robinson, J.Am.Chem.Soc. 89 (1967), 181; S. Sakakibara, Y. Shimonishi, Bull.Chem.Soc.Japan 38 (1965), 1412; E. Atherton et al., J.Chem.Soc.Perkin Trans.I 1981, 538; S.S. Wang, J.Am.Chem.Soc. 95 (1973), 1328) durchgeführt. So erhält man z.B. aus 8 nach Deprotonierung und Kupplung mit Boc-Leucin das gebundene Dipeptid 9 (Beispiel 4). 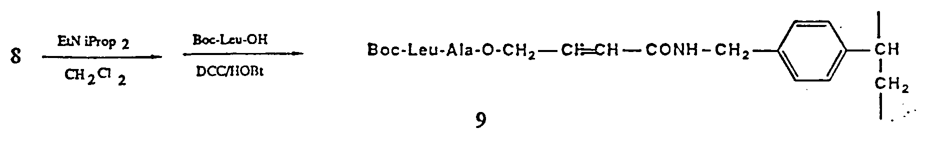
Durch Fixierung von Boc-Isoleucin am erfindungsgemäßen polymeren Träger, wie vom Typ 5, und durch zwei anschließende Synthesecyclen, bestehend in N-terminaler Schutzgruppenabspaltung, Freilegung der terminalen Aminogruppe und Kettenverlängerung a) mit N²-tert-Butyloxycarbonyl-N⁴(2-acetamido-3,4,6-tri-O-acetyl-2-desoxy-β-D-glucopyranosyl)asparagin und b) mit Boc-Leucin wurde das Glycotripeptid 10 in festphasengebundener Form synthetisiert (Beispiel 5). 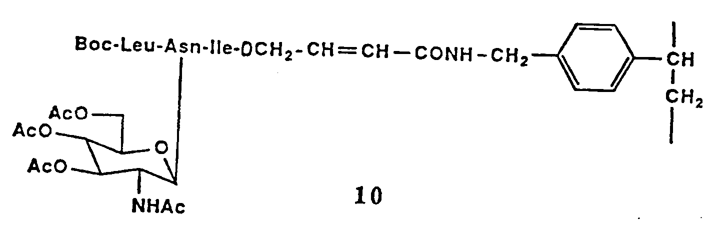
Als weiteres veranschaulichendes Beispiel wurde nach dem erfindungsgemäßen Verfahren der Peptidwirkstoff Leu-Enkephalin in festphasengebundener Form 11 aufgebaut (Beispiel 6).

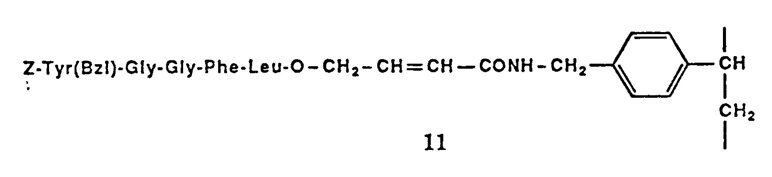

### Die Abspaltung der Peptide und Glycopeptide vom erfindungsgemäßen Träger

Das besondere Merkmal der erfindungsgemäßen polymeren Träger und der damit durchgeführten Festphasenpeptidsynthesen ist die Anbindung der Aminosäuren, Peptide und Glycopeptide als allylische Ester (z.B. in 8, 9, 10, 11). Daraus ergibt sich als entscheidender Vorteil, daß die synthetisierten Peptide und Glycopeptide unter sehr milden, praktisch neutralen Bedingungen vom Träger abgespalten werden können. Man erreicht diese Abspaltung z.B. durch Behandlung der polymergebundenen Derivate mit katalytischen Mengen eines Palladium-(O)-Katalysators unter Sauerstoffausschluß in Tetrahydrofuran oder anderen Lösungsmittelsystemen in Gegenwart eines geeigneten Nucleophils, wie Morpholin, Dimedon oder einer anderen leicht deprotonierbaren CH-aciden Verbindung. Die Reaktion ist bei Raumtemperatur in der Regel in wenigen Stunden vollständig abgelaufen. Durch die außerordentlich milden Abspaltungsbedingungen bleiben selbst empfindliche Strukturen (z.B. die glycosidischen Bindungen) und Schutzgruppen (wie z.B. die Boc- und Z-Schutzgruppen) im abgelösten Peptid (z.B. 12, 14) oder Glycopeptid (z.B. 13) zuverlässig erhalten. 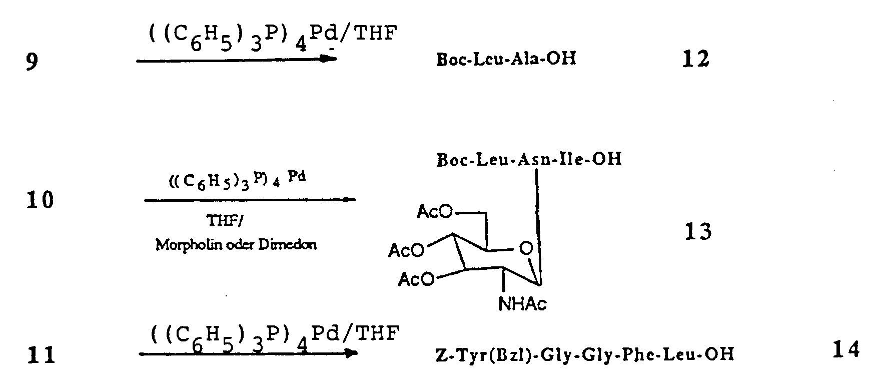
Die abgelösten Produkte, wie z.B. 12 (Beispiel 7), 13 (Beispiel 8) und 14 (Beispiel 9), können dadurch selektiv und partiell geschützt in guten Ausbeuten isoliert werden. Sie sind deshalb sofort zu weiteren Synthesen, wie gezielten Fragmentkondensationen, einsetzbar. Sie können natürlich auch von den restlichen Schutzgruppen befreit werden.
Die erfindungsgemäßen Träger, wie z.B. 5, die die Festphasensynthese von Peptiden und Glycopeptiden über als (substituierte) Allylester an den Träger gebundene Bausteine ermöglichen, haben damit entscheidende Vorteile:
Sie sind einfach abzubauen und
erlauben eine effektive, racemisierungsfreie Ankupplung des C-terminalen Bausteins. Die Festphasenpeptidsynthese kann mit säure- und basenlabilen Aminoschutzgruppen durchgeführt werden. Die Abspaltung der synthetisierten Peptide oder Glycopeptide ist unter nahezu neutralen Bedingungen sehr effektiv möglich; dabei werden empfindliche Strukturelemente, wie z.B. Glycosidbindungen und Säure- (z.B. Boc) und basenlabile (z.B. OAc) Schutzgruppen, nicht angegriffen.

Die nachfolgenden Beispiele sollen die Erfindung näher erläutern, ohne ihren Umfang darauf einzuschränken.

### Beispiel 1 Synthese eines aktivierten bzw. aktivierbaren polymeren Trägers mit allylischen Ankergruppen der allgemeinen Formel I:

### N-(4-Brom-crotonoyl)-aminomethyl-polystyrol 5

2 g Aminomethyl-polystyrol 3 (0,8 m Äquiv. NH₂/g Harz) werden in 40 ml absol.Dichlormethan mit 1,02 g (6.2 mmol) 4-Bromcrotonsäure, o,2 g (1,65 mmol) 4-Dimethylaminopyridin und 1,53 g (7,5 mmol) N,N′-Dicyclohexylcarbodiimid 12 h bei Raumtemp. gerührt. Dann wird das Polymer über eine D3-Fritte abfiltriert, mit Dichlormethan, Methanol, Dimethylformamid und wiederum Dichlormethan gewaschen und i.Hochvak. bei 30°C getrocknet.
Das polymere Material zeigt im IR-Spektrum diejenigen Banden, die die Umsetzung zum Produkt (5) mit der allylischen Ankergruppe anzeigen:
IR(KBr):ν(cm⁻¹)= 3300 (NH); 1670 (Amid I). 970 (C-CH=CH-).

### Beispiel 2 Kupplung einer C-terminalen Aminosäure an den polymeren Träger;

### N-tert.-Butyloxycarbonyl-L-alanyl-(4-hydroxycrotonoylamidomethyl)-polystyrol 7 (N-Boc-Ala-"Hycram"-polystyrol)

1,59 mmol der N-geschützten Aminosäure (im Beispiel 2 tert-Butyloxycarbonyl-L-alanin) werden in 30 ml Methanol mit 0,26 g (0,79 mmol) Cäsiumcarbonat 2 h bei Raumtemp. gerührt. Dann wird das Lösungsmittel abgedampft und vom verbleibenden Cäsiumsalz (hier 6) mehrmals absol. Toluol abdestilliert. Das so gewonnene Cäsiumsalz wird nun mit 0,9 g des nach Beispiel 1 hergestellten polymeren Trägers mit der allylischen Ankergruppe 24 h in 50 ml Dimethylformamid (DMF) gerührt. Dann wird das entstandene Kupplungsprodukt 7 abfiltriert, mit DMF, Dioxan und Dichlormethan gewaschen und i.Vak. bei 30-35°C getrocknet.
Das Hycram-(=4-Hydroxycrotonoylamidomethyl)polystyrol-gebundene Boc-Alanin zeigt im IR-Spektrum die zusätzlichen IR-Banden:
IR(KBr):ν(cm⁻¹)= 1740 (C=o,Ester); 1710-1700 (c=o, Urethan).
Die folgenden beiden Beispiele zeigen den typischen Reaktionscyclus, der bei Peptid- und Glycopeptidsynthesen an erfindungsgemäßen Trägersystemen mit allylischen Ankergruppen bei jeder Kettenverlängerung durchgeführt wird.

### Beispiel 3 Abspaltung der N-terminalen Schutzgruppe; Abspaltung der tert -Butyloxycarbonylgruppe vom Hycram-polystyrol-gebundenen Boc-Alanin, Alanyl-Hycram-polystyrol 8

Das nach Beispiel 2 synthetisierte Boc-Alanyl-Hycram-Polystyrol 7 (1,3 g) wird mit 20 ml 5o % Trifluoressigsäure in Dichlormethan 1,5 h geschüttelt. Danach wird abfiltriert, 4 mal mit absol. Dichlormethan geschüttelt und abfiltriert. Das so erhaltene Hydro-trifluoracetat des polymergebundenen Alanins 8 wird zweimal mit 0,5 ml Ethyl-diisopropylamin in 10 ml Dichlormethan je 10 min geschüttelt und abfiltriert und schließlich zweimal mit absol. Dichlormethan geschüttelt und filtriert.
Im IR-Spektrum des Produkts 8 ist die typische Urethan-Bande (ν= 1710-1700 cm⁻¹) nicht mehr vorhanden.

### Beispiel 4 Kettenverlängerung eines über eine allylische Ankergruppe an den polymeren Träger gebundenen Aminoacyl- oder Peptid-Bausteins.

### Boc-Leucyl-alanyl-Hycram-Polystyrol 9

1,02 g des nach Beispiel 3 hergestellten polymergebundenen Alanins 8 werden in 30 ml absol. Dichlormethan mit 0,87 g (3,7 mmol) Boc-Leucin, 0,93 g (4,5 mmol) Dicyclohexylcarbodiimid und 0,1 g (0,75 mmol) 1-Hydroxybenzotriazol 12 h bei Raumtemp. geschüttelt.

Das polymergebundene Dipeptid 9 wird abfiltriert, mit Dichlormethan, Dimethylformamid und wiederum Dichlormethan gewaschen und getrocknet.

### Beispiel 5 Synthese eines N-Glycopeptids am polymeren Träger: Boc-Leucyl-[N⁴-(2-acetamido-3,4,6-tri-O-acetyl-2-desoxy-β-D-glucopyranosyl)]asparaginyl-isoleucyl-Hycram-Polystyrol 10

Entsprechend der in den Beispielen 2-4 beschriebenen Vorgehensweise wird zunächst aus 0,32 g Bromcrotonoylamidomethylpolystyrol 5 und 0,32 g (1,38 mmol) Boc-Isoleucin über das Cäsiumsalz das Boc-L-Isoleucyl-Polymer hergestellt. Dieses wird mit Trifluoressigsäure/Dichlormethan N-terminal deblockiert. Mit 40 ml 5 proz. Ethyl-diisopropylamin in Dichlormethan wird die terminale Ammoniumgruppe deprotoniert.
Die Kettenverlängerung wird mit N²-tert-Butyloxycarbonyl-[N⁴-(2-acetamido-3,4,6-tri-O-acetyl-2-desoxy-β-D-glucopyranosyl)]-L-asparagin (0,34 g = 0,6 mmol) analog zu Beispiel 4 ausgeführt. Die N-terminale Deblockierung und die Kettenverlängerung wird nun entsprechend mit tert -Butyloxycarbonyl-L-leucin (0,43 g= 1,85 mmol) wiederholt. Alle Wasch- und Trocknungsschritte werden entsprechend der Vorschrift in Beispiel 4 ausgeführt. Man erhält so das über die allylische Ankergruppe an das Polymer gebundene Glycotripeptid 10.

### Beispiel 6: Synthese von terminal geschütztem Leu-Enkephalin am polymeren Träger; Z-Tyr(Bzl)-Gly-Gly-Phe-Leu-Hycram-Polystyrol 11

Nach den in den Beispielen 2-4 beschriebenen Schritten werden zunächst 1,41 g (3,9 mmol) Boc-Leucin über das Cäsiumsalz mit 1 g 4-Brom-crotonoylamido (BCA)-Polystyrol 5 (Beispiel 1) umgesetzt. Vom erhaltenen Kupplungsprodukt wird mit 50 proz. Trifluoressigsäure in Dichlormethan die Boc-Schutzgruppe abgespalten. Das terminal deblockierte, an Hycram-Polystyrol gebundene Leucin wird nun mit 1,03 g (3,9 mmol) Boc-Phenylalanin in Gegenwart von 0,96 g (4,7 mmol) Dicyclohexylcarbodiimid und 0,1 g (0,74 mmol) 1-Hydroxybenzotriazol in 30 ml Dichlormethan verknüpft.
Es schließen sich weitere Synthesecyclen aus Boc-Gruppen-Abspaltung, Deprotonierung und Kupplung mit zweimal 0,68 g (3,9 mmol) Boc-Glycin, und 1,57 (3,9 mmol) Z-(O-Benzyl)tyrosin an, wobei die Kupplungen jeweils mit den gleichen Mengen Dicyclohexylcarbodiimid und 1-Hydroxybenzotriazol in diesen drei Cyclen in 30 ml absol. Dimethylformamid ausgeführt werden. Das gewonnene erfindungsgemäß polymergebundene Pentapeptid 11 wird dann zur Ablösung vom Polymer eingesetzt (Beispiel 9).

### Beispiel 7 Abspaltung des Peptids vom erfindungsgemäßen polymeren Träger : tert -Butyloxycarbonyl-L-leucyl-L-alanin 12

Das an Hycram-Polystyrol gebundene Boc-Leucyl-alanin 9 (hergestellt nach Beispiel 4) (1,15 g) wird in 50 ml sauerstoffreiem Tetrahydrofuran unter Stickstoff mit 4 ml Morpholin und 0,1 g Tetrakis(triphenylphosphino)palladium-(O) 2 h bei Raumtemp.
geschüttelt. Das Harz wird abfiltriert, mehrmals mit Dichlormethan und mit Methanol gewaschen. Die vereinigten organischen Lösungen werden i.Vak. von den Lösungsmitteln befreit, der Rückstand in 70 ml Dichlormethan aufgenommen und mehrmals mit 20 proz. Zitronensäure ausgeschüttelt. Die organische Phase wird dann dreimal mit 30 ml gesättigter Natriumbicarbonat-Lösung extrahiert, die wäßrige Phase auf pH 1,5 gebracht und mit 6 mal 30 ml Dichlormethan ausgeschüttelt. Die vereinigten Dichlormethanlösungen werden über Na₂SO₄ getrocknet und i.Vak. von Lösungsmittel befreit. Als Rückstand verbleiben 130 mg tert-Butyloxycarbonyl-L-leucyl-L-alanin 12 ( = 80 % der maximal gewinnbaren Menge bezogen auf die reaktiven 4-Brom-crotonoylamido-Gruppen am Harz 5) R_{f} = 0.15 (Chloroform/Methanol 5:1).
400 MHz-¹H-NMR(CDCl₃): δ = 7.13 (m, 1H,NH,Ala); 5.35 (m,1H,NH,Urethan); 4.53 (m,1H,α-CH,Ala); 4.22 (m,1H,α-CH,Leu); 1.75-1.53(m,3H,CH₂-CH(CH₃)₂,Leu); 1.40 (m,12H, CH₃ von Boc,Ala); 0.95-0.83 (m,6H,CH₃,Leu).

### Beispiel 8 Abspaltung eines Glycopeptids vom polymeren Träger: N-tert-Butyloxycarbonyl-L-leucyl-[N⁴-(2-acetamido-3,4,6-tri-O-acetyl-2-desoxy-β-D-glucopyranosyl)]-L-asparaginyl-L-isoleucin 13

0,5 g des polymergebundenen Glycopeptids 10 werden unter Stickstoff mit 2 ml Morpholin und 0,1 g Tetrakis (triphenylphosphino)palladium-(O) in 30 ml Tetrahydrofuran 12 h geschüttelt. Man filtriert das Harz ab und wäscht mit Tetrahydrofuran und Methanol nach. Die vereinigten organischen Lösungen werden i.Vak. eingedampft, der Rückstand wird in Dichlormethan/methanol (12/1) über Kieselgel chromatographiert. Man erhält 70 mg (= 50 % der maximal gewinnbaren Menge) des Glycotripeptids 13. R_{f}= 0.29 (Dichlormethan/methanol 5:1).
400 MHz-¹H-NMR (Dimethylsulfoxid-d₆) δ = 8.72 (d,J=10Hz, 1H,β-NH,Asn); 8,32 (m,1H, α-NH,Asn); 7.99 (d,breit, J=10Hz,1H,NH-Ac); 7.08 (m,1H,NH,Ile); 6.9 (d, J=10Hz, 1H,NH,Urethan,Leu; 5.25-5.11 (m,2H,H-1,H-3); 4.85-4.75 (m,1H,H-4); 4.62 (m, 1H, α-CH,Asn); 4.25-4.10 (m,2H,H-6a, α-CH-Ile); 4.03-3.87 (m,2H,H-6b, H-2 oder H-5);2.75 (m,1H,β-CH₂,Asn): 2.25 (m,1H,β-CH₂,Asn); 2,0 (s,3H, AcO,Zucker); 1.95 (s,3H,AcO,Zucker); 1.92 (s,3H,AcO,Zucker); 1.73 (s breit,4H,AcNH,Zucker,β-CH,Ile); 1.65-1.52 (m, 2H,CH₂Leu), 1.34 (s breit, 11H, CH₃ von Boc, CH₂-CH ,Leu, 1HCH₂-CH₃,Ile); 1.1-0.95 (m,1H,1H von CH₂-CH₃Ile); 0.88 (m,12H,CH₃ von Leu (2x) und Ile (2x)).

### Beispiel 9 Abspaltung eines Peptidwirkstoffs vom erfindungsgemäßen polymeren Träger:

### Geschütztes Leu-Enkephalin 14

1,7 g des polymergebundenen N-terminal Z- und O-Benzylethergeschützten Leu-Enkephalins 11 werden unter Stickstoff mit 10 ml Morpholin und 0,1 g Tetrakis(triphenylphosphino)palladium-(O) 10 h in 50 ml Tetrahydrofuran geschüttelt. Dann wird vom Polymeren abfiltriert, mit Tetrahydrofuran und Methanol gründlich gewaschen, und die vereinigten organischen Lösungen werden i.Vak. eingedamft.
Die Reinigung erfolgt durch mehrmaliges Umfällen aus Chloroform/Ether, wobei das Produkt als Gel durch Abzentrifugieren gewonnen wird. Als Ausbeute erhält man 150 mg des geschützten Leu-Enkephalins. (= 33 % der maximal gewinnbaren Ausbeute).
400 MHz-¹H-NMR (Dimethylsulfoxid-d₆):
δ= 8.5 (d,J=8Hz,1H,NH); 8.39 (m,1H,NH,Gly); 8.17 (d,J=8Hz,1H,NH); 8.08 (M,1H,NH,Gly);7.54 (d,J=8Hz,NH,Tyr); 7.46-7.05 (m,17H,Aromat:Phe,Z,Bzl,2H von Tyr); 6.88 (d,J=10Hz,2H,2H von Tyr); 5.03 (s,2H,CH₂ von Z); 4.96-4.84 (m,2H,CH₂ von Bzl); 4.53 (m,1H, α-CH,Leu); 4.29-4.14 (m,2H, α-CH,Tyr; α-CH,Phe); 2.82-2.62 (m,2H,CH₂,Thyr); 1.65-1.54 (m,2H,CH₂Leu); 1.48 (m,1H,-CH-CH₃,Leu); 0.88 (d,J=7Hz, 3H,CH₃,Leu); 0.82 (d,J=7Hz,3H,CH₃,Leu).

## Patentansprüche (Patentansprüche für folgende(n) Vertragsstaat(en): AT, BE, CH, DE, FR, GB, GR, IT, LI, LU, NL, SE)

1. Allylische Seitenketten enthaltendes Festphasensystem, in dem die allylischen Seitenketten an ein festes Trägermaterial gebunden sind, mit der allgemeinen Formel 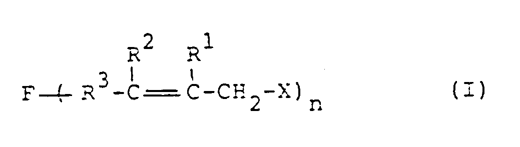 worin F ein festes Trägermaterial bedeutet, R¹ und R² gleich oder verschieden sind und Wasserstoff, eine Alkylgruppe mit 1 bis 7 Kohlenstoffatomen, eine substituierte oder unsubstituierte ein- oder zweikernige Arylgruppe oder Halogen bedeuten und zwei Alkylsubstituenten zusammen mit einer Arylgruppe in R¹ oder R² auch ein System aus 2 oder mehreren Ringen bilden können, R³ eine Verknüpfungsgruppierung (Spacer) bedeutet, X Hydroxyl, Halogen, eine Sulfonatgruppe, eine Phosphonatgruppe oder eine Acyloxygruppe bedeutet, wobei Acyl in der Acyloxygruppe den Rest einer aliphatischen Carbonsäure mit 1 bis 7 Kohlenstoffatomen darstellt oder den Rest RCO- darstellt, der den geschützten oder ungeschützten Rest einer Aminosäure, eines Peptids, Glycopeptids, Nucleotids, einer Hydroxycarbonsäure, einer Dicarbonsäure oder einer Tricarbonsäure bedeutet, der über die Carbonylgruppe gebunden ist, und n die Zahl der auf dem Trägermaterial gebundenen Seitenketten ist.

2. Festphasensystem nach Anspruch 1,
**dadurch gekennzeichnet,**
daß das feste Trägermaterial ein organisches oder anorganisches Polymeres ist.

3. Festphasensystem nach Anspruch 1 oder 2,
**dadurch gekennzeichnet,**
daß das feste Trägermaterial ein festes Basismaterial ist, das mit einem für die Verknüpfung mit den Allylseitenketten geeigneten Material überzogen ist.

4. Festphasensystem nach Anspruch 2 oder 3,
**dadurch gekennzeichnet,**
daß das Polymere oder das für die Verknüpfung mit den Allylseitenketten geeignete Material ein vernetztes Polystyrol ist.

5. Festphasensystem nach Anspruch 4,
**dadurch gekennzeichnet,**
daß das Polystyrol eine Aminomethylgruppe trägt.

6. Festphasensystem nach Anspruch 5,
**dadurch gekennzeichnet,**
daß es an den Aminomethylgruppen die Gruppierung
-CO-(CH₂)ₓ-C(R²)=C(R¹)-CH₂-X
trägt, worin X die in Anspruch 1 angegebene Bedeutung besitzt und x 0 oder eine ganze Zahl von 1 bis 7 bedeutet.

7. Verfahren zur Herstellung von Verbindungen der allgemeinen Formel I nach einem der Ansprüche 1 bis 6, worin X Hydroxyl, Halogen, eine Sulfonatgruppe, eine Phosphonatgruppe oder eine Acyloxygruppe in der Bedeutung eines Säurerestes einer aliphatischen Carbonsäure ist,
**dadurch gekennzeichnet,**
daß man ein festes Trägermaterial, das zur Verknüpfung mit der Allylgruppierung -CR²=CR¹-CH₂-X geeignete funktionelle Gruppen A enthält, mit einer Verbindung B-CR²=CR¹-CH₂-X umsetzt, worin A und B Atomgruppierungen bedeuten, die unter Kondensation und/oder Addition und Ausbildung einer Verknüpfung zwischen festem Trägermaterial und Allylgruppierung miteinander reagieren.

8. Verfahren nach Anspruch 7,
**dadurch gekennzeichnet,**
daß das feste, funktionelle Gruppen A enthaltende Trägermaterial ein aminomethyliertes Polystyrol ist, das mit einer terminal allylisch substituierten ungesättigten Carbonsäure oder einem Carbonsäurederivat, oder mit den 1,3-Allylisomeren davon, unter Bildung einer Amidgruppierung umgesetzt wird.

9. Verfahren nach Anspruch 8,
**dadurch gekennzeichnet,**
daß die allylisch substituierte Carbonsäure 4-Halogen-, 4-Hydroxy-, 4-Acyloxy- oder 4-Sulfonyloxy-Crotonsäure ist.

10. Verfahren zur Herstellung von Verbindungen der allgemeinen Formel I nach einem der Ansprüche 1 bis 6, worin X eine Acyloxygruppe bedeutet, in der der Acylrest RCO- den geschützten oder ungeschützten Rest einer Aminosäure, eines Peptids, eines Glycopeptides, eines Nucleotides, oder einer Hydroxycarbonsäure, einer Di- oder Tricarbonsäure bedeutet,
**dadurch gekennzeichnet,**
daß man ein Festphasensystem der allgemeinen Formel (I), worin X Hydroxyl, Halogen, eine Sulfonatgruppe, eine Phosphonatgruppe oder eine Acyloxygruppe, worin Acyl den Rest einer aliphatischen Carbonsäure darstellt, bedeutet, mit N-geschützten Aminosäuren, Peptiden, Glykopeptiden, Nucleotiden, Hydroxycarbonsäuren, Dicarbonsäuren oder Tricarbonsäuren, oder Derivaten oder Salzen davon, umsetzt und gegebenenfalls die N-terminalen Schutzgruppen abspaltet.

11. Verfahren zur Synthese von ungeschützten oder partiell geschützten Peptiden, Glycopeptiden, Nucleotiden, Hydroxycarbonsäuren, Dicarbonsäuren oder Tricarbonsäuren,
**dadurch gekennzeichnet,**
daß man aus einer Verbindung der allgemeinen Formel (I), worin X eine Acyloxygruppe bedeutet, in der der Acylrest RCO- den geschützten oder ungeschützten Rest eines Peptids, Glycopeptids, Nucleotids, einer Hydroxycarbonsäure, einer Dicarbonsäure oder einer Tricarbonsäure bedeutet, den Acylrest RCO in Gegenwart katalytischer Mengen einer Palladiumverbindung abspaltet.

12. Verwendung eines Festphasensystems der allgemeinen Formel (I) nach einem der Ansprüche 1 bis 6, worin X Hydroxyl, Halogen, eine Sulfonatgruppe, eine Phosphonatgruppe oder eine Acyloxygruppe, worin Acyl den Rest einer aliphatischen Carbonsäure darstellt, bedeutet, für Festphasenreaktionen.

13. Verwendung nach Anspruch 12,
**dadurch gekennzeichnet,**
daß die Festphasenreaktion eine Festphasensynthese von Peptiden, Glycopeptiden, Nucleotiden oder Proteinen ist.

## Patentansprüche (Patentansprüche für folgende(n) Vertragsstaat(en): ES)

1. Verfahren zur Herstellung eines allylische Seitenketten enthaltenden Festphasensystems, in dem die allylischen Seitenketten an ein festes Trägermaterial gebunden sind, mit der allgemeinen Formel 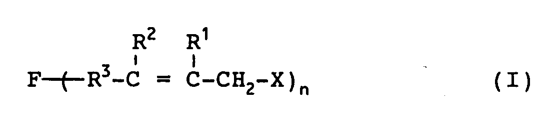 worin F ein festes Trägermaterial bedeutet, R¹ und R² gleich oder verschieden sind und Wasserstoff, eine Alkylgruppe mit 1 bis 7 Kohlenstoffatomen, eine substituierte oder unsubstituierte ein- oder zweikernige Arylgruppe oder Halogen bedeuten und zwei Alkylsubstituenten zusammen mit einer Arylgruppe in R¹ oder R² auch ein System aus 2 oder mehreren Ringen bilden können, R³ eine Verknüpfungsgruppierung (Spacer) bedeutet, X Hydroxyl, Halogen, eine Sulfonatgruppe, eine Phosphonatgruppe oder eine Acyloxygruppe bedeutet, wobei Acyl in der Acyloxygruppe den Rest einer aliphatischen Carbonsäure mit 1 bis 7 Kohlenstoffatomen darstellt und n die Zahl der auf dem Trägermaterial gebundenen Seitenketten ist,
**dadurch gekennzeichnet,**
daß man ein festes Trägermaterial, das zur Verknüpfung mit der Allylgruppierung -CR²=CR¹-CH₂-X geeignete funktionelle Gruppen A enthält, mit einer Verbindung B-CR²=CR¹-CH₂-X umsetzt, worin A und B Atomgruppierungen bedeuten, die unter Kondensation und/oder Addition und Ausbildung einer Verknüpfung zwischen festem Trägermaterial und Allylgruppierung miteinander reagieren.

2. Verfahren nach Anspruch 1,
**dadurch gekennzeichnet,**
daß das feste, funktionelle Gruppen A enthaltende Trägermaterial ein aminomethyliertes Polystyrol ist, das mit einer terminal allylisch substituierten ungesättigten Carbonsäure oder einem Carbonsäurederivat, oder mit den 1,3-Allylisomeren davon, unter Bildung einer Amidgruppierung umgesetzt wird.

3. Verfahren nach Anspruch 2,
**dadurch gekennzeichnet,**
daß die allylisch substituierte Carbonsäure 4-Halogen-, 4-Hydroxy-, 4-Acyloxy- oder 4-Sulfonyloxy-Crotonsäure ist.

4. Verfahren zur Herstellung eines allylische Seitenketten enthaltenden Festphasensystems, in dem die allylischen Seitenketten an ein festes Trägermaterial gebunden sind, mit der allgemeinen Formel 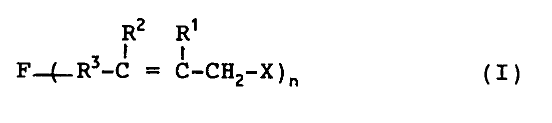 worin F ein festes Trägermaterial bedeutet, R¹ und R² gleich oder verschieden sind und Wasserstoff, eine Alkylgruppe mit 1 bis 7 Kohlenstoffatomen, eine substituierte oder unsubstituierte ein- oder zweikernige Arylgruppe oder Halogen bedeuten und zwei Alkylsubstituenten zusammen mit einer Arylgruppe in R¹ oder R² auch ein System aus 2 oder mehreren Ringen bilden können, R³ eine Verknüpfungsgruppierung (Spacer) bedeutet, X den Rest RCO- darstellt, der den geschützten oder ungeschützten Rest einer Aminosäure, eines Peptids, Glycopeptids, Nucleotids, einer Hydroxycarbonsäure, einer Dicarbonsäure oder einer Tricarbonsäure bedeutet, der über die Carbonylgruppe gebunden ist, und n die Zahl der auf dem Trägermaterial gebundenen Seitenketten ist,
**dadurch gekennzeichnet,**
daß man ein Festphasensystem der allgemeinen Formel (I), worin X Hydroxyl, Halogen, eine Sulfonatgruppe, eine Phosphonatgruppe oder eine Acyloxygruppe, worin Acyl den Rest einer aliphatischen Carbonsäure darstellt, bedeutet, mit N-geschützten Aminosäuren, Peptiden, Glykopeptiden, Nukleotiden, Hydroxycarbonsäuren, Dicarbonsäuren oder Tricarbonsäuren, oder Derivaten oder Salzen davon, umsetzt und gegebenenfalls die N-terminalen Schutzgruppen abspaltet.

5. Verfahren nach einem der Ansprüche 1 bis 4,
**dadurch gekennzeichnet,**
daß das feste Trägermaterial ein organisches oder anorganisches Polymeres ist.

6. Verfahren nach einem der Ansprüche 1 bis 5,
**dadurch gekennzeichnet,**
daß das feste Trägermaterial ein festes Basismaterial ist, das mit einem für die Verknüpfung mit den Allylseitenketten geeigneten Material überzogen ist.

7. Verfahren nach Anspruch 5 oder 6,
**dadurch gekennzeichnet,**
daß das Polymere oder das für die Verknüpfung mit den Allylseitenketten geeignete Material ein vernetztes Polystyrol ist.

8. Verfahren nach Anspruch 7,
**dadurch gekennzeichnet,**
daß das Polystyrol eine Aminomethylgruppe trägt.

9. Verfahren nach Anspruch 8,
**dadurch gekennzeichnet,**
daß an den Aminomethylgruppen die Gruppierung
-CO-(CH₂)ₓ-C(R²)=C(R¹)-CH₂-X
vorliegt, worin X die in den Ansprüchen 1 oder 4 angegebene Bedeutung besitzt und x 0 oder eine ganze Zahl von 1 bis 7 bedeutet.

10. Verfahren zur Synthese von ungeschützten oder partiell geschützten Peptiden, Glycopeptiden, Nucleotiden, Hydroxycarbonsäuren, Dicarbonsäuren oder Tricarbonsäuren,
**dadurch gekennzeichnet,**
daß man aus einer Verbindung der allgemeinen Formel (I), worin X eine Acyloxygruppe bedeutet, in der der Acylrest RCO- den geschützten oder ungeschützten Rest eines Peptids, Glycopeptids, Nucleotids, einer Hydroxycarbonsäure, einer Dicarbonsäure oder einer Tricarbonsäure bedeutet, den Acylrest RCO in Gegenwart katalytischer Mengen einer Palladiumverbindung abspaltet.

11. Verwendung eines Festphasensystems der allgemeinen Formel (I), worin X Hydroxyl, Halogen, eine Sulfonatgruppe, eine Phosphonatgruppe oder eine Acyloxygruppe, worin Acyl den Rest einer aliphatischen Carbonsäure darstellt, bedeutet, für Festphasenreaktionen.

12. Verwendung nach Anspruch 11,
**dadurch gekennzeichnet,**
daß die Festphasenreaktion eine Festphasensynthese von Peptiden, Glycopeptiden, Nucleotiden oder Proteinen ist.

## Claims (Claims for the following Contracting State(s): AT, BE, CH, DE, FR, GB, GR, IT, LI, LU, NL, SE)

1. Allylic side chain-containing solid phase system, in which the allylic side chains are bound to a solid carrier material, with the general formula 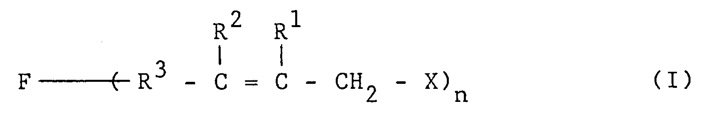 wherein F signifies a solid carrier material, R¹ and R² are the same or different and signify hydrogen, an alkyl group with 1 to 7 carbon atoms, a substituted or unsubstituted mono- or dinuclear aryl group or halogen and two alkyl substituents, together with an aryl group in R¹ or R², can also form a system of 2 or more rings, R³ signifies a linking grouping (spacer), X signifies hydroxyl, halogen, a sulphonate group, a phosphonate group or an acyloxy group, whereby acyl in the acyloxy group represents the residue of an aliphatic carboxylic acid with 1 to 7 carbon atoms or the radical RCO-, which signifies the protected or unprotected residue of an amino acid, of a peptide, glycopeptide, nucleotide, of a hydroxycarboxylic, of a dicarboxylic acid or of a tricarboxylic acid which is bound via the carbonyl group and n is the number of the side chains bound to the carrier material.

2. Solid phase system according to claim 1, characterised in that the solid carrier material is an organic or inorganic polymer.

3. Solid phase system according to claim 1 or 2, characterised in that the solid carrier material is a solid base material which is covered with a material suitable for the linking with the allyl side chains.

4. Solid phase system according to claim 2 or 3, characterised in that the polymer or the material suitable for the linking with the allyl side chains is a cross-linked polystyrene.

5. Solid phase system according to claim 4, characterised in that the polystyrene carries an aminomethyl group.

6. Solid phase system according to claim 5, characterised in that it carries on the aminomethyl groups the grouping
-CO(̵CH₂)ₓ-C(R²)=C(R¹)-CH₂-X
wherein X possesses the meaning given in claim 1 and x signifies 0 or a whole number from 1 to 7.

7. Process for the preparation of compounds of the general formula (I) according to one of claims 1 to 6, wherein X is hydroxyl, halogen, a sulphonate group, a phosphonate group or an acyloxy group in the meaning of an acid residue of an aliphatic carboxylic acid, characterised in that one reacts a solid carrier material, which contains functional groups A suitable for the linking with the allyl grouping -CR²=CR¹-CH₂-X, with a compound B-CR²=CR¹-CH₂-X, wherein A and B signify atom groupings which react with one another with condensation and/or addition and formation of a linking between solid carrier material and the allyl grouping.

8. Process according to claim 7, characterised in that the solid carrier material containing the functional groups A is an aminomethylated polystyrene which is reacted with a terminally allylic-substituted unsaturated carboxylic acid or a carboxylic acid derivative or with the 1,3-allyl isomers thereof with formation of an amide grouping.

9. Process according to claim 8, characterised in that the allylic-substituted carboxylic acid is 4-halo-, 4-hydroxy-, 4-acyloxy- or 4-sulphonyloxy-crotonic acid.

10. Process for the preparation of compounds of the general formula I according to one of claims 1 to 6, wherein X signifies an acyloxy group in which the acyl radical RCO- signifies the protected or unprotected residue of an amino acid, of a peptide, or a glycopeptide, of a nucleotide or of a hydroxycarboxylic, of a di- or tricarboxylic acid, characterised in that one reacts a solid phase system of the general formula (I), wherein X signifies hydroxyl, halogen, a sulphonate group, a phosphonate group or an acyloxy group, wherein acyl represents the residue of an aliphatic carboxylic acid, with N-protected amino acids, peptides, glycopeptides, nucleotides, hydroxycarboxylic acids, dicarboxylic acids or tricarboxylic acids or derivatives or salts thereof, and possibly splits off the N-terminal protective groups.

11. Process for the synthesis of unprotected or partly protected peptides, glycopeptides, nucleotides, hydroxycarboxylic acids, dicarboxylic acids and tricarboxylic acids, characterised in that, from a compound of the general formula (I), wherein X signifies an acyloxy group, in which the acyl radical RCO- signifies the protected or unprotected residue of a peptide, glycopeptide, nucleotide, of a hydroxycarboxylic acid, of a dicarboxylic acid or of a tricarboxylic acid, one splits off the acyl radical RCO- in the presence of catalytic amounts of a platinum metal compound.

12. Use of a solid phase system of the general formula (I) according to one of claims 1 to 6, wherein X represents hydroxyl, halogen, a sulphonate group, a phosphonate group or an acyloxy group, wherein acyl represents the residue of an aliphatic carboxylic acid, for solid phase reactions.

13. Use according to claim 12, characterised in that the solid phase reaction is a solid phase synthesis of peptides, glycopeptides, nucleotides or proteins.

## Claims (Claims for the following Contracting State(s): ES)

1. Process for the preparation of an allylic side chain-containing solid phase system in which the allylic side chains are bound to the solid carrier material with the general formula 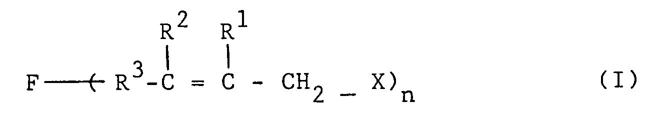 wherein F signifies a solid carrier material, R¹ and R² are the same or different and signify hydrogen, an alkyl group with 1 to 7 carbon atoms, a substituted or unsubstituted mono- or dinuclear aryl group or halogen and two alkyl substituents, together with an aryl group in R¹ or R², can also form a system of 2 or more rings, R³ signifies a linking grouping (spacer), X signifies hydroxyl, halogen, a sulphonate group, a phosphonate group or an acyloxy group, whereby acyl in the acyloxy group represents the residue of an aliphatic carboxylic acid with 1 to 7 carbon atoms and n is the number of the side chains bound to the carrier material, characterised in that one reacts a solid carrier material, which contains functional groups A suitable for the linking with the allyl grouping -CR²=CR¹-CH₂-X, with a compound B-CR²=CR¹-CH₂-X, wherein A and B signify atom groupings which react with one another with condensation and/or addition and formation of a linking between solid carrier material and allyl grouping.

2. Process according to claim 1, characterised in that the solid, functional group A-containing carrier material is an aminomethylated polystyrene which is reacted with a terminally allylic-substituted unsaturated carboxylic acid or a carboxylic acid derivative or with the 1,3-allyl isomers thereof with formation of an amide grouping.

3. Process according to claim 2, characterised in that the allylic-substituted carboxylic acid is 4-halo-, 4-hydroxy-, 4-acyloxy- or 4-sulphonyloxy-crotonic acid.

4. Process for the preparation of an allylic side chain-containing solid phase system in which the allylic side chains are bound to a solid carrier material with the general formula 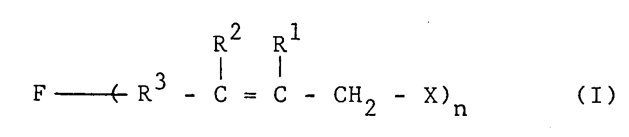 wherein F signifies a solid carrier material, R¹ and R² are the same or different and signify hydrogen, an alkyl group with 1 to 7 carbon atoms, a substituted or unsubstituted mono- or binuclear aryl group or halogen and two alkyl substituents, together with an aryl group in R¹ or R², can also form a system of 2 or more rings, R³ signifies a linking grouping (spacer), X represents the radical RCO- which is bound to the protected or unprotected residue of an amino acid, of a peptide, glycopeptide, nucleotide, of a hydroxycarboxylic acid, of a dicarboxylic acid or of a tricarboxylic acid which is bound via the carbonyl group and n is the number of the side chains bound to the carrier material, characterised in that one reacts a solid phase system of the general formula (I), wherein X represents hydroxyl, halogen, a sulphonate group, a phosphonate group or an acyloxy group, wherein acyl represents the residue of an aliphatic carboxylic acid, with N-protected amino acids, peptides, glycopeptides, nucleotides, hydroxycarboxylic acids, dicarboxylic acids or tricarboxylic acids or derivatives or salts thereof and possibly splits off the N-terminal protective group.

5. Process according to one of claims 1 to 4, characterised in that the solid carrier material is an organic or inorganic polymer.

6. Process according to one of claims 1 to 5, characterised in that the solid carrier material is a solid base material which is coated with a material suitable for the linking with the allyl side chains.

7. Process according to claim 5 or 6, characterised in that the polymer or the material suitable for the linking with the allyl side chains is a cross-linked polystyrene.

8. Process according to claim 7, characterised in that the polystyrene carries an aminomethyl group.

9. Process according to claim 8, characterised in that, on the aminomethyl group, the grouping
-CO-(CH₂)ₓ-C(R²)=C(R¹)-CH₂-X
is present, wherein X possesses the meaning given in claims 1 or 4 and x signifies 0 or a whole number from 1 to 7.

10. Process for the synthesis of unprotected or partly protected peptides, glycopeptides, nucleotides, hydroxycarboxylic acids, dicarboxylic acids or tricarboxylic acids, characterised in that, from a compound of the general formula (I), wherein X signifies an acyloxy group in which the acyl radical RCO- signifies the protected or unprotected residue of a peptide, glycopeptide, nucleotide, of a hydroxycarboxylic acid, of a dicarboxylic acid or of a tricarboxylic acid, one splits off the acyl radical RCO in the presence of catalytic amounts of a palladium compound.

11. Use of a solid phase system of the general formula (I), wherein X signifies hydroxyl, halogen, a sulphonate group, a phosphonate group or an acyloxy group, wherein acyl represents the residue of an aliphatic carboxylic acid, for solid phase reactions.

12. Use according to claim 11, characterised in that the solid phase reaction is a solid phase synthesis of peptides, glycopeptides, nucleotides or proteins.

## Revendications (Revendications pour l'(les) Etat(s) contractant(s) suivant(s): AT, BE, CH, DE, FR, GB, GR, IT, LI, LU, NL, SE)

1. Système en phase solide contenant des chaînes latérales allyliques, dans lequel les chaînes latérales allyliques sont liées à un support solide, et qui a la formule générale 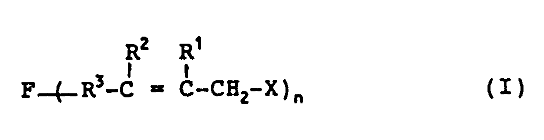 dans laquelle F est un support solide, R¹ et R² sont identiques ou différents et représentent l'hydrogène, un groupe alkyle de 1 à 7 atomes de carbone, un groupe aryle mono- ou bicyclique substitué ou non substitué ou un halogène, et deux substituants alkyle peuvent aussi former ensemble, avec un groupe aryle dans R¹ ou R², un système de 2 ou plusieurs cycles, R³ est un groupe de liaison (espaceur), X représente un hydroxyle, un halogène, un groupe sulfonate, un groupe phosphonate ou un groupe acyloxy, l'acyle dans le groupe acyloxy représentant le reste d'un acide carboxylique aliphatique de 1 à 7 atomes de carbone ou le reste RCO- qui représente le reste protégé ou non protégé d'un aminoacide, d'un peptide, d'un glycopeptide, d'un nucléotide, d'un acide hydroxycarboxylique, d'un acide dicarboxylique ou d'un acide tricarboxylique qui est lié par l'intermédiaire du groupe carbonyle, et n est le nombre de chaînes latérales liées au support.

2. Système en phase solide selon la revendication 1, caractérisé en ce que le support solide est un polymère organique ou inorganique.

3. Système en phase solide selon la revendication 1 ou 2, caractérisé en ce que le support solide est une matière de base solide qui est revêtue d'une matière appropriée pour la liaison avec les chaînes latérales allyliques.

4. Système en phase solide selon la revendication 2 ou 3, caractérisé en ce que le polymère ou la matière appropriée pour la liaison avec les chaînes latérales allyliques est un polystyrène réticulé.

5. Système en phase solide selon la revendication 4, caractérisé en ce que le polystyrène porte un groupe aminométhyle.

6. Système en phase solide selon la revendication 5, caractérisé en ce qu'il porte sur les groupes aminométhyle le groupement
-CO-(CH₂)ₓ-C(R²)=C(R¹)-CH₂-X
dans lequel X possède la signification donnée dans la revendication 1 et x est 0 ou un nombre entier de 1 à 7.

7. Procédé de préparation de composés de formule générale I selon l'une des revendications 1 à 6, dans laquelle X est un hydroxyle, un halogène, un groupe sulfonate, un groupe phosphonate ou un groupe acyloxy au sens d'un reste d'acide d'un acide carboxylique aliphatique, caractérisé en ce que l'on fait réagir un support solide qui contient des groupes fonctionnels A aptes à former une liaison avec le groupement allylique -CR²=CR¹-CH₂X, avec un composé B-CR²=CR¹-CH₂X, où A et B représentent des groupements d'atomes qui réagissent entre eux par condensation et/ou addition et formation d'une liaison entre le support solide et le groupement allylique.

8. Procédé selon la revendication 7, caractérisé en ce que le support solide contenant des groupes fonctionnels A est un polystyrène aminométhylé que l'on fait réagir avec un acide carboxylique ou un dérivé d'acide carboxylique insaturé substitué en position terminale par un groupe allylique, ou avec leurs isomères 1,3-allyliques, en formant un groupement amide.

9. Procédé selon la revendication 8, caractérisé en ce que l'acide carboxylique à substitution allylique est un acide 4-halogéno-, 4-hydroxy-, 4-acyloxy- ou 4-sulfonyloxycrotonique.

10. Procédé de préparation de composés de formule générale I selon l'une des revendications 1 à 6, dans laquelle X est un groupe acyloxy dans lequel le reste acyle RCO- représente le reste protégé ou non protégé d'un aminoacide, d'un peptide, d'un glycopeptide, d'un nucléotide ou d'un acide hydroxycarboxylique, d'un acide di- ou tricarboxylique, caractérisé en ce que l'on fait réagir un système en phase solide de formule générale (I) dans laquelle X est un hydroxyle, un halogène, un groupe sulfonate, un groupe phosphonate ou un groupe acyloxy où acyle est le reste d'un acide carboxylique aliphatique, avec des aminoacides, peptides, glycopeptides, nucléotides, acides hydroxycarboxyliques, acides dicarboxyliques ou acides tricarboxyliques N-protégés, ou des dérivés ou sels de ces acides, et éventuellement on élimine les groupes protecteurs N-terminaux.

11. Procédé de synthèse de peptides, glycopeptides, nucléotides, acides hydroxycarboxyliques, acides dicarboxyliques ou acides tricarboxyliques, non protégés ou partiellement protégés, caractérisé en ce que l'on sépare, en présence de quantités catalytiques d'un composé du palladium, le reste acyle RCO d'un composé de formule générale (I) dans laquelle X représente un groupe acyloxy, dans lequel le reste acyle RCO- représente le reste protégé ou non protégé d'un peptide, d'un glycopeptide, d'un nucléotide, d'un acide hydroxycarboxylique, d'un acide dicarboxylique ou d'un acide tricarboxylique.

12. Utilisation d'un système en phase solide de formule générale (I) selon l'une des revendications 1 à 6, dans lequel X est un hydroxyle, un halogène, un groupe sulfonate, un groupe phosphonate ou un groupe acyloxy où acyle est le reste d'un acide carboxylique aliphatique, pour des réactions en phase solide.

13. Utilisation selon la revendication 12, caractérisée en ce que la réaction en phase solide est une synthèse en phase solide de peptides, de glycopeptides, de nucléotides ou de protéines.

## Revendications (Revendications pour l'(les) Etat(s) contractant(s) suivant(s): ES)

1. Procédé de préparation d'un système en phase solide contenant des chaînes latérales allyliques, dans lequel les chaînes latérales allyliques sont liées à un support solide, et qui a la formule générale 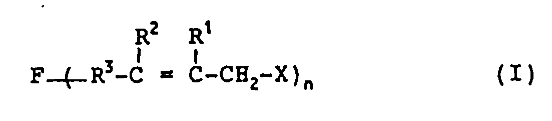 dans laquelle F est un support solide, R¹ et R² sont identiques ou différents et représentent l'hydrogène, un groupe alkyle de 1 à 7 atomes de carbone, un groupe aryle mono- ou bicyclique substitué ou non substitué ou un halogène, et deux substituants alkyle peuvent aussi former ensemble, avec un groupe aryle dans R¹ ou R², un système de 2 ou plusieurs cycles, R³ est un groupe de liaison (espaceur), X représente un hydroxyle, un halogène, un groupe sulfonate, un groupe phosphonate ou un groupe acyloxy, l'acyle dans le groupe acyloxy représentant le reste d'un acide carboxylique aliphatique de 1 à 7 atomes de carbone, et n est le nombre de chaînes latérales liées au support, caractérisé en ce que l'on fait réagir un support solide qui contient des groupes fonctionnels A aptes à former une liaison avec le groupement allylique -CR²=CR¹-CH₂X, avec un composé B-CR²=CR¹-CH₂X, où A et B représentent des groupements d'atomes qui réagissent entre eux par condensation et/ou addition et formation d'une liaison entre le support solide et le groupement allylique.

2. Procédé selon la revendication 1, caractérisé en ce que le support solide contenant des groupes fonctionnels A est un polystyrène aminométhylé que l'on fait réagir avec un acide carboxylique ou un dérivé d'acide carboxylique insaturé substitué en position terminale par un groupe allylique, ou avec leurs isomères 1,3-allyliques, en formant un groupement amide.

3. Procédé selon la revendication 2, caractérisé en ce que l'acide carboxylique à substitution allylique est un acide 4-halogéno-, 4-hydroxy-, 4-acyloxy- ou 4-sulfonyloxycrotonique.

4. Procédé de préparation d'un système en phase solide contenant des chaînes latérales allyliques, dans lequel les chaînes latérales allyliques sont liées à un support solide, et qui a la formule générale 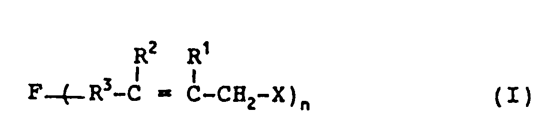 dans laquelle F est un support solide, R¹ et R² sont identiques ou différents et représentent l'hydrogène, un groupe alkyle de 1 à 7 atomes de carbone, un groupe aryle mono- ou bicyclique substitué ou non substitué ou un halogène, et deux substituants alkyle peuvent aussi former ensemble, avec un groupe aryle dans R¹ ou R², un système de 2 ou plusieurs cycles, R³ est un groupe de liaison (espaceur), X représente le reste RCO- qui représente le reste protégé ou non protégé d'un aminoacide, d'un peptide, d'un glycopeptide, d'un nucléotide, d'un acide hydroxycarboxylique, d'un acide dicarboxylique ou d'un acide tricarboxylique qui est lié par l'intermédiaire du groupe carbonyle, et n est le nombre de chaînes latérales liées au support, caractérisé en ce que l'on fait réagir un système en phase solide de formule générale (I) dans laquelle X est un hydroxyle, un halogène, un groupe sulfonate, un groupe phosphonate ou un groupe acyloxy où acyle est le reste d'un acide carboxylique aliphatique, avec des aminoacides, peptides, glycopeptides, nucléotides, acides hydroxycarboxyliques, acides dicarboxyliques ou acides tricarboxyliques N-protégés, ou des dérivés ou sels de ces acides, et éventuellement on élimine les groupes protecteurs N-terminaux.

5. Procédé selon l'une des revendications 1 à 4, caractérisé en ce que le support solide est un polymère organique ou inorganique.

6. Procédé selon l'une des revendications 1 à 5, caractérisé en ce que le support solide est une matière de base solide qui est revêtue d'une matière appropriée pour la liaison avec les chaînes latérales allyliques.

7. Procédé selon la revendication 5 ou 6, caractérisé en ce que le polymère ou la matière appropriée pour la liaison avec les chaînes latérales allyliques est un polystyrène réticulé.

8. Procédé selon la revendication 7, caractérisé en ce que le polystyrène porte un groupe aminométhyle.

9. Procédé selon la revendication 8, caractérisé en ce que, sur les groupes aminométhyle, se trouve le groupement
-CO-(CH₂)ₓ-C(R²)=C(R¹)-CH₂-X
dans lequel X possède la signification donnée dans les revendications 1 ou 4 et x est 0 ou un nombre entier de 1 à 7.

10. Procédé de synthèse de peptides, glycopeptides, nucléotides, acides hydroxycarboxyliques, acides dicarboxyliques ou acides tricarboxyliques, non protégés ou partiellement protégés, caractérisé en ce que l'on sépare, en présence de quantités catalytiques d'un composé du palladium, le reste acyle RCO d'un composé de formule générale (I) dans laquelle X représente un groupe acyloxy, dans lequel le reste acyle RCO- représente le reste protégé ou non protégé d'un peptide, d'un glycopeptide, d'un nucléotide, d'un acide hydroxycarboxylique, d'un acide dicarboxylique ou d'un acide tricarboxylique.

11. Utilisation d'un système en phase solide de formule générale (I), dans laquelle X est un hydroxyle, un halogène, un groupe sulfonate, un groupe phosphonate ou un groupe acyloxy où acyle est le reste d'un acide carboxylique aliphatique, pour des réactions en phase solide.

12. Utilisation selon la revendication 11, caractérisée en ce que la réaction en phase solide est une synthèse en phase solide de peptides, de glycopeptides, de nucléotides ou de protéines.
